# EUROPEAN PATENT SPECIFICATION

(11) **EP 3 399 878 B1**
(45) Date of publication and mention of the grant of the patent: **25.11.2026**
(21) Application number: 16816164.4
(22) Date of filing: 01.07.2016
(51) Int. Cl.: A41D 13/05, A41D 13/06, A41D 13/08, A41D 13/018

(54) **PROTECTION SYSTEM AND METHOD**
SCHUTZSYSTEM UND -VERFAHREN
SYSTÈME ET PROCÉDÉ DE PROTECTION

(30) Priority: 04.01.2016 CN 201610006866
(43) Date of publication of application: 14.11.2018
(73) Proprietor: BOE Technology Group Co., Ltd., Beijing 100015 (CN)
(72) Inventor: YUAN, Zuo, Beijing 100176 (CN); ZHANG, Yifei, Beijing 100176 (CN)
(74) Representative: Cohausz & Florack
(86) International application number: PCT/CN2016/088169
(87) International publication number: WO 2017/117934

(56) References cited:
- EP-A2- 1 247 698
- WO-A2-2012/104833
- CN-A- 102 307 627
- CN-A- 102 961 833
- CN-A- 104 366 752
- CN-A- 105 495 772
- CN-U- 204 908 025
- DE-A1- 102013 213 306
- DE-A1- 4 003 683
- US-A- 5 500 952
- US-A1- 2014 047 623
- US-A1- 2014 123 374
- US-A1- 2015 101 112

## Description

### TECHNICAL FIELD

The present disclosure relates to a protection system and method.

### BACKGROUND

Elderly people may have decreased visual acuity and reduced ability to respond to falling, and some may also suffer from diseases such as osteoporosis and muscle atrophy. As a result, they may easily fall to the ground, and often incur severe injuries.

Upon falling, protruding parts of a human body, such as knees, elbows, and other joints, are prone to injuries. Some protect gears may be worn by those more susceptible to such injuries, with a focus on protecting the protruding body parts. Such devices may include wrist bracers, knee pads, and other protective gears, inside which may include internal cushion layers with elasticity and functions to cushion the impact

For such protective gears not to severely limit a user's mobility, the internal cushion layer is typically designed not to be thick, which results in limited cushioning, and thus insufficient protection to the user.

US patent application US 2014/123374 A1 discloses a protective gear for cushioning a part of a human body or an object against an impact, comprising a housing conforming to a portion of the part of a human body or the object; an airbag disposed in the housing; a detector and a controller. The document discloses a fall-protection system. The system has at least one sensor for sensing if the person is falling and for outputting a status signal. The system also has a processor configured for receiving the status signal from the at least one sensor, and for outputting a corresponding command signal. The system also has a support for the at least one sensor and the processor, the support being portable by the person. The system also has a fall-protection device mountable to the support and configured for receiving the command signal from the processor. The fall-protection device has inflatable membranes, at least some of which are deployable as elongated extensions so to protect a part of the person by forming a substantially flower-like enclosure before said part contacts the ground surface. The fall-protection device is deployable upon the processor outputting a command signal indicative that the person is falling.

### SUMMARY

The invention is set out in the appended set of claims.

### BRIEF DESCRIPTION OF DRAWINGS

To more clearly illustrate some of the embodiments, the following is a brief description of the drawings. The drawings in the following descriptions are only illustrative of some embodiments. For those of ordinary skill in the art, other drawings of other embodiments can become apparent based on these drawings.
FIG. 1 is a schematic diagram of the three-dimensional structure of a protective gear according to some embodiments of the disclosure;
FIG. 2 is a cross-sectional view of the protective gear according to some embodiments of the disclosure;
FIG. 3 is a schematic diagram of the structure of a deployed airbag provided in the protective gear;
FIG. 4 is a schematic diagram of an internal structure of an airbag slot provided in the protective gear;
FIG. 5 is a structural diagram of a protective gear system according to some embodiments of the disclosure;
FIG. 6 is a flowchart of a method for fall protection according to some embodiments of the disclosure.

### DETAILED DESCRIPTION

In the following, with reference to the drawings of the embodiments disclosed herein, the technical solutions of the embodiments of the invention will be described in a clear and fully understandable way. It is noted that the described embodiments are merely a portion but not all of the embodiments of the invention. Based on the described embodiments of the invention, those ordinarily skilled in the art can obtain other embodiment(s), which come(s) within the scope sought for protection by the invention.

This disclosure provides a protective gear and a method of use thereof, for cushioning an object against an impact The object is a part of a human body or a delicate device. The impact is that generated upon falling on the ground. There is no limitation herein.

In the following, description of the protective gear and the method of use thereof primarily uses an example of a human body against falling on the ground.

### Embodiment 1:

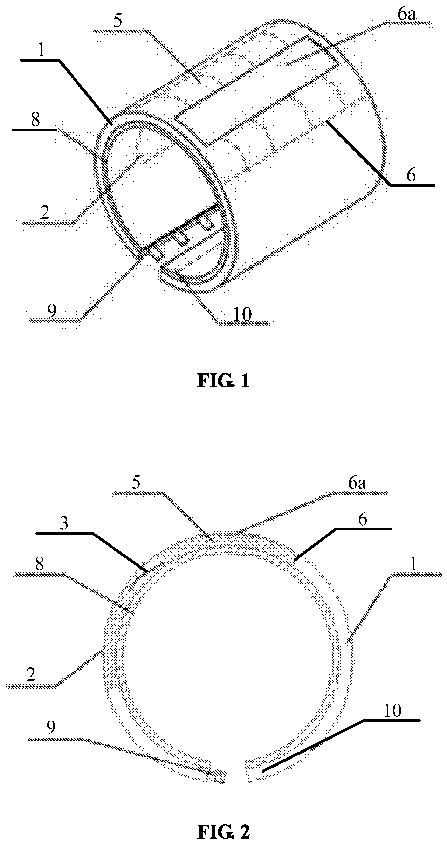

This embodiment of the disclosure provides a protective gear, as shown in FIGS. 1 and 2. The protective gear includes a housing 1, an acceleration sensor, a controller, a gas generator, an airbag 5, and an airbag groove 6. The airbag groove 6 is arranged in the housing 1, and is provided with an airbag outlet, arranged on a side of the airbag groove 6 that is close to an outer surface of the housing 1. The gas generator and the airbag 5 are disposed in the airbag groove 6. The acceleration sensor and the controller are arranged in the housing 1. The acceleration sensor, the controller, and the gas generator are sequentially coupled. It is noted that the term "an outer surface of the housing 1" means the surface opposite to human body when the protective gear is worn on the human body, and correspondingly, the surface of the housing 1 that is close to the human body is referred an inner surface of the housing 1.

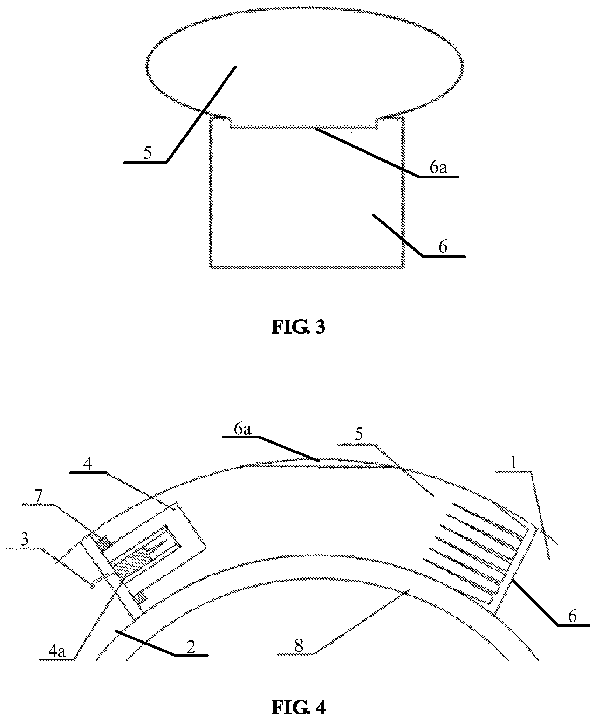

The process in which the above protective gear protects the human body is as follows: the protective gear is worn on the body such that the airbag outlet is arranged on a side of the protective gear corresponding to a protruding part of a bent joint, in order to ensure that after ejection from the airbag groove 6, the airbag 5 is right at, and thereby capable of protecting, the protruding part of the joint while bending. The acceleration sensor detects acceleration along the X-axis, Y-axis and Z-axis of the body in a real-time manner, and the detected acceleration data are then sent to the controller. Compared with during normal walking, acceleration along the X-axis, Y-axis and Z-axis changes during falling, thus based on the acceleration data, the controller can determine whether falling occurs, and if it determines that falling happens, the controller generates an actuating signal, which is further transmitted to the gas generator. After the gas generator receives the actuating signal, it rapidly produces a large amount of gas to inflate, or pop up, the airbag 5. Rapid expansion of the airbag 5 causes the airbag to be ejected or popped out from the airbag outlet 6a. The airbag 5 that has been inflated and ejected is illustrated in FIG. 3. Because the airbag outlet is arranged on the side of the airbag groove 6 that is close to the outer surface of the housing 1, the airbag 5 is located on an outside of the housing 1 after popping up, thus can provide protection to the human body by cushioning the impact of the ground to the human body when it is about to impact the ground during falling.

It should be noted that the "X-axis, Y-axis and Z-axis" shall be referenced in a three-dimensional coordinate system during the movement of a human body. The two mutually perpendicular axes in a horizontal plane are defined as the "X axis" and the "Y-axis", and the axis perpendicular to the horizontal plane and across the intersection of the X axis and the Y axis is defined as the "Z-axis."

Because the cushioning effect of the airbag 5 is much larger than the cushion layer typically used in conventional protective gears, the protective gear disclosed herein provides protection to a falling body by arranging the airbag 5 in the protective gear.

In addition, since the airbag 5 is located in the airbag groove 6 under normal situations, and will be inflated and popped out of the airbag groove 6 only when falling occurs, so the airbag 5 occupies little space in the protective gear. The acceleration sensor, controller and gas generators are all relatively small, so these parts do not lead to an increased thickness and weight for the protective gear.

In the protective gear as shown in FIG. 4, a gas generator 4 may include a tip discharge device 4a and a gas generating agent. The tip discharge device 4a is coupled to the controller, and after receiving the actuating signal transmitted by the controller, the tip discharge device 4a can generate an electrical spark. The gas generating agent is a substance that under fire or heat will have a violent reaction such that a large amount of gas is quickly produced. Thus upon exposure of the electrical spark generated by the tip discharge device 4a, the gas generating agent quickly produces a large amount of gas, which goes into and inflates the airbag 5. A gas generating agent can be a non-azide gas generant or liquid CO₂. Furthermore, the gas generator may also include a shell 4, configured to accommodate the tip discharge device 4a and the gas generating agent The shell is configured to be permeable to gas, so that the gas generated by the reaction of the gas generating agent can get into the airbag 5.

To facilitate maintenance and/or replacement, the airbag 5 can be detachably mounted in the airbag groove 6, and as such a retractable window can be arranged on a side wall of the airbag groove 6 that is closer to the outer surface of the housing 1. When it is required for replacement/maintenance of the airbag 5, the retractable window on the airbag groove can be opened to dismount, or detach, the airbag 5. There are multiple approaches to detachably mount the airbag 5. As shown in FIG. 4, an air inlet of the airbag 5 can be mounted on the side wall of airbag groove 6 via threads 7. On the basis of this structure, in order to facilitate the gas to go into and inflate the airbag 5, the gas generator 4 can be also installed on the side wall of the airbag groove 6 at a position located at the air inlet of the airbag 5.

If the volume of the airbag 5 is too small after full expansion, the cushioning effect is limited, and airbag 5 cannot effectively protect the human bodies. If the volume of the airbag 5 is too large after full expansion, inflation time may be too long for a complete expansion before falling on the ground, affecting the protective effects of the protective gear. Thus the volume of the airbag 5 after full expansion is preferably controlled within a reasonable range. Assuming a constant inflation speed, the volume of the airbag 5 after full expansion depends on inflation time, and in order to fully expand the airbag 5 before falling, the inflation time should be within the time period from when the human body loses balance to when the body falls to the ground. As such the time period from when the airbag starts inflation to when it is fully expanded is preferably less than or equal to 200 ms, so that the volume of the fully expanded airbag 5 is controlled within a reasonable range. In some embodiments, a fully expanded airbag 5 can have a length of 15-20 cm and a width of 5-10 cm, to ensure that the air bag 5 can have a sufficient cushioning effect. Herein the length refers to a diameter of the airbag that is typically perpendicular to the direction of the impact; and the width, or thickness, refers to a diameter of the airbag along the direction of the impact

In order to increase the integration level of all parts inside the protective gear, and to reduce the space occupied by these parts, the acceleration sensor and the controller can be integrated on a circuit board 2 in some embodiments, as shown in FIG. 4. The circuit board 2 can be arranged on one side of the airbag groove 6. The gas generator 4 can be coupled with the circuit board 2 via a wire 3, configured to realize an electrical coupling between the gas generator 4 and the controller of the circuit board 2. In order to reduce the length of the wire 3, the circuit board 2 can be arranged at a proximity of the gas generator 4.

The protective gears provided in this embodiment may take a ring-shaped structure, which can be worn around parts, such as elbows, knees etc. of the human body. In order to achieve a wearable function of the protective gear as shown in FIGS. 1 and 2, the protective gear can be disassembled at a side opposite to the side of the airbag groove 6. Among the two ends of the dissembled protective gear, one end can provided with plugs 9, and the other end can be provided with sockets 10 which correspondingly matches with the plugs 9, and by snap-fit or securely connection, of the plugs 9 with the sockets 10, the protective gear can be worn on the human body. Other means may be arranged on the protective gear to play a similar role to allow the wearing of the protective gear on the human body.

In order to further improve the protective effect of the protective gear, a cushion layer 8 may be arranged at an inside surface of the housing 1, as shown in FIGS. 1, 2 and 4. The cushion layer 8 can be made of an elastic material such as sponge. When a human body falls, the cushion layer 8 can have elastic deformation to cushion the impact of the ground to the body, to thereby play a protective role.

In some embodiments, the housing 1 of the protective gear can be made of a material such as a hard plastic or a metal, which is configured to prevent friction between the body and the ground when falling occurs.

The protective gear as described above can be used to protect elbows, knees and other parts of the body. Take a protective gear for elbow as an example. When wearing the protective gear, the airbag outlet shall be arranged to be on a side corresponding to the protruding part of a bent elbow, to ensure that the airbag 5 ejected and popped up from the airbag outlet 6a can be located on the protruding side of the bent elbow. This configuration ensures that when a body is about to impact the ground after falling, the airbag is right on the protruding side of a bent elbow, which can cushion the impact of the ground to the protrusion part of the elbow. A knee protective gear is worn and works in a similar manner.

### Embodiment 2

On the basis of Embodiment 1, Embodiment 2 provides a protective gear system, which comprises a primary protective gear, at least one secondary protective gear, and at least one wireless communication module. The primary protective gear is the protective gear as described above in Embodiment 1. Compared with the protective gear as described in Embodiment 1, a secondary protective gear lacks an acceleration sensor and a controller, and includes a housing, an airbag groove, a gas generator and an airbag, whose specifications can be referenced by the housing 1, the airbag groove 6, the gas generator 4 and the airbag 5, as disclosed in Embodiment 1. Both the primary protective gear and each of the at least one secondary protective gear are provided with a wireless communication module; the wireless communication module of the primary protective gear is coupled to a controller of the primary protective gear, whereas the wireless communication module of the secondary protective gear is coupled to the gas generator in the secondary protective gear.

The acceleration sensor of the primary protective gear detects acceleration of human body along the X-axis, Y-axis and Z-axis in a real-time manner. When falling occurs, based on the acceleration data of the human body along the X-axis, Y-axis and Z-axis detected by the acceleration sensor, the controller of the primary protective gear determines whether falling occurs, and if it determines that falling happens, the controller generates an actuating signal, and further sends the actuating signal to the gas generator 4. The gas generator rapidly produces a large amount of gas to inflate and expand the airbag 5. At the same time, via the wireless communication module of the primary protective gear, the actuating signal is transmitted to the wireless communication module of the secondary protective gear, which further sends an actuating signal to the gas generator 4 arranged in the secondary protective gear, so that the air bag 5 is inflated and expanded, to thereby play protective roles of the primary protective gear and the secondary protective gear.

In the protective gear system described above, airbags 5 are configured to play a cushioning function to a human body against the impact of the ground when falling occurs. Since airbags 5 have a larger cushioning effect than the cushion layer commonly used in current protective gears, the protective gear system has an improved protection.

Additionally in this embodiment, only one set of acceleration sensor and controller is arranged on one, but not the rest, of a plurality of protective gears in the protective gear system, and the plurality of protective gears are able to communicate with one another through the wireless communication modules. By this configuration, one protective gear can simultaneously control the airbags 5 in multiple protective gears to expand, without the need to arrange acceleration sensors and controllers in each of the plurality of protective gears, thereby leading to a saving on the number of acceleration sensors and controllers. Thus the cost of the protective gear system is reduced and its structure is simplified.

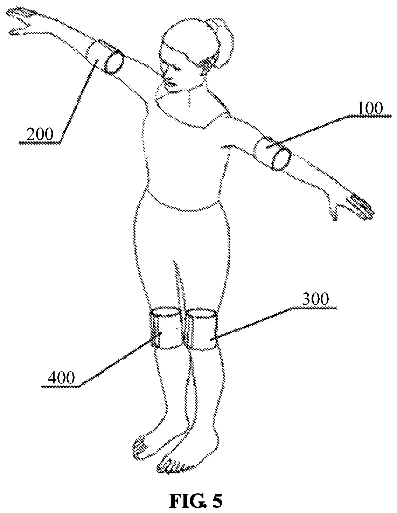

In some embodiments, the protective gear system may include two protective gears for elbows (or elbow protective gears) and two protective gears for knees (or knee protective gears). Any one of the four protective gears can be the primary protective gear and the remaining three the secondary protective gears. In one embodiment as shown in FIG. 5, the protective gear system includes a primary elbow protective gear 100, a secondary elbow protective gear 200, a secondary knee protective gear 300, and a secondary knee protective gear 400. Each of the protective gears is provided with a wireless communication module. When the primary elbow protective gear 100 detects that falling occurs, its controller controls its airbag 5 to expand, and transmits an actuating signal to the secondary elbow protective gear 200, the secondary knee protective gear 300 and the secondary knee protective gear 400, respectively, to expand their respective airbags 5.

It should be noted that, for the above-mentioned protective gear system, in order to ensure the airbags 5 to be able to cushion the body when falling, the airbag outlets 6a are preferably arranged on a side corresponding to the protruding side of human joints during wearing of the protective gear system. Specifically for the primary elbow protective gear 100 and the secondary elbow protective gear 200, because the elbow joints protrude toward the rear side of the body when they are bent, when wearing they should be configured such that their airbag outlets are arranged to point to the rear side of the body. For the secondary knee protective gear 300 and the secondary knee protective gear 400, because the knee joints protrude toward the front of the body when they are bent, when wearing they should be configured such that their airbag outlets are arranged to point to the front of the body.

The protective gear system as described in this embodiment can also include protective gears for other parts of the body, such as head, neck, waist, etc., so as to provide more comprehensive protection to a human body when falling.

### Embodiment 3

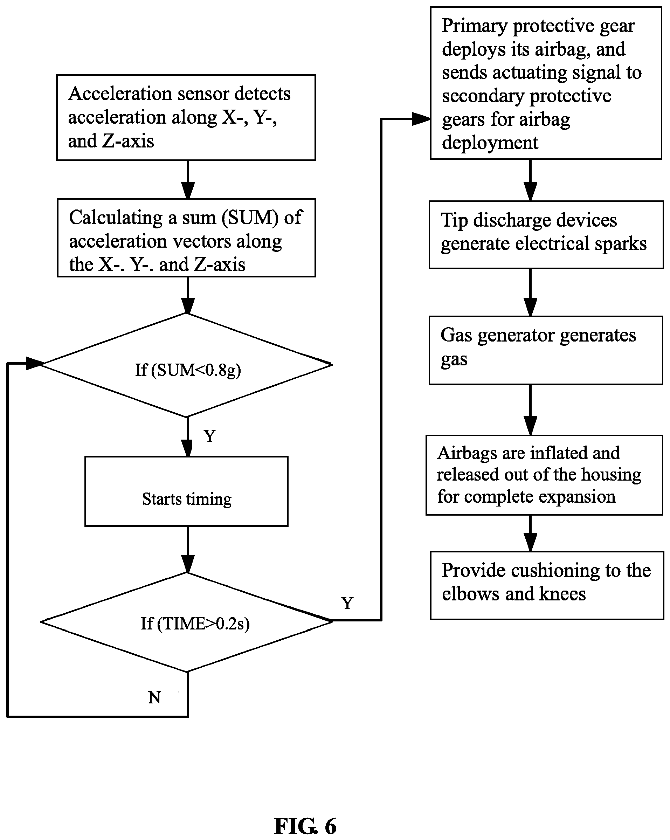

In this embodiment, a method for fall protection is provided. The method for fall protection is applied to a protective gear as described in Embodiment 1, and a protective gear system as described in Embodiment 2. As shown in FIG. 6, the method for fall protection comprises:
Step S1: detecting acceleration of a human body along the X-axis, Y-axis and Z-axis in a real-time manner;
Step S2: determining whether falling occurs, and proceeding to step S3 if determining that falling occurs;
Step S3: controlling the gas generator 4 to generate gas, wherein the gas inflates the airbag 5 to allow the airbag 5 to be ejected from the airbag outlet 6a.

This embodiment utilizes airbags 5 to provide protection when a human body falls. Additionally, the method for fall protection as described above is to detect acceleration of the human body along the X-axis, Y-axis and Z-axis in a real-time manner, and determine whether falling occurs. When falling happens, the airbag 5 is inflated and expanded automatically, which can cushion the impact of the ground to the human body, thereby realizing an intelligent and automatic fall protection.

In some embodiments of the method for fall protection as described above, also as shown in FIG. 6, step S2 includes the following steps:
Step S21: calculating a sum of the acceleration vectors along the X-axis, Y-axis and Z-axis of a human body;
Step S22: determining if the sum of the acceleration vectors along the X-axis, Y-axis and Z-axis of the human body is less than or equal to a threshold value of the sum of the acceleration vectors, and if yes, starting timing and proceeding to step S23;
Step S23: determining within a continuous preset time period, if the sum of the acceleration vectors along the X-axis, Y-axis and Z-axis of the human body is less than or equal to the threshold value of the sum of the acceleration vectors, and if yes, determining that fall happens.

It should be noted that the "threshold value of the sum of the acceleration vectors" should be less than the maximum value for the sum of acceleration vectors along the X-axis, Y-axis and Z-axis of the human body, to ensure an accuracy in determining if falling occurs. After actual calculation, when falling happens accidentally, the sum of the acceleration vector along the X-axis, Y-axis, and Z-axis of a human body is typically less than 1 g (g is the gravitational acceleration, having a value of 9.8 m/s2), and thus the threshold value of the sum of the acceleration vectors should be set to be smaller than 1 g.

In addition, the "preset time period" should be less than the whole time period of falling, so as to ensure an accuracy in determining if falling occurs. When falling occurs, the human body instantly loses balance, and the whole falling process is essentially the process in which the body's gravity center falls down from a certain height to the ground. The whole time period of falling is set as t, the reduced height of the body's gravity center is set as h, and based on $h = \frac{1}{2} {gt}^{2}$, and a given h, the whole time period of falling t can be calculated. Assuming h = 1 m, t can be 452 ms, and as such the "preset time period" should be set to be less than 452 ms.

In the whole falling process, the sum of acceleration vectors along the X-axis, Y-axis and Z-axis gradually reduces to zero, thus when the sum of acceleration vectors is initially detected to be less than the threshold value of the sum of the acceleration vectors, it indicates that falling may likely occur, and timing starts from here, and the acceleration along the X-axis, the Y-axis and the Z-axis is continuously detected. If the sum of acceleration vectors along the X-axis, the Y-axis and the Z-axis becomes less than the threshold value of the sum of the acceleration vectors within the preset time period, the probability of falling becomes very high, it is determined that falling occurs.

To improve the judging accuracy and prevent automatic pop-up and expansion of airbags 5 under false judgement, a large number of tests have been performed to optimize the threshold value for the sum of acceleration vectors, and it is observed that a threshold value in a range of 0.6-0.9 g corresponds to a relatively high judging accuracy, and more specifically of 0.8 g to the highest.

To improve the judging accuracy and allow sufficient time for timely inflation and complete expansion of airbags 5, a large number of tests have been performed to optimize the preset time period, and it is observed that a preset time period in a range of 100-300 ms corresponds to a relatively high judging accuracy, and more specifically of 200 ms to the highest.

Regardless of normal walking or falling, detection of acceleration along the X-axis, Y-axis and Z-axis by the acceleration sensor is performed in a real-time manner. The more frequently the acceleration sensor detects, the more the judgement to determine if the sum of acceleration vector along the X-axis, Y-axis and Z-axis of a human body is less than or equal to the threshold value within the preset time period, and the higher the judging accuracy. In the above embodiments, the detection frequency of the acceleration sensor can be greater than or equal to 20/s, i.e., for a preset time period of 200 ms, the acceleration sensor needs to detect at least 4 times to guarantee a high judging accuracy in determining whether falling happens.

It should be noted that, the protective gear requires to be worn such that the airbag outlets are arranged on a side of the protective gears corresponding to the protruding part of the body joints in order for the airbags 5 to sufficiently cushion the impact of the ground to the body upon falling. This configuration ensures that after ejection from the airbag outlets, the airbags 5 is right beneath the protrusion parts of the bent joints.

## Claims

1. A protective gear for cushioning a part of a human body or an object against an impact, the impact happening after falling of the part of a human body or the object to ground, the protective gear comprising:
a housing (1) conforming to a portion of the part of a human body or the object;
an airbag (5) disposed in the housing (1);
a detector comprising an acceleration sensor configured to detect acceleration vectors of the part of a human body or the object along an X-axis, a Y-axis and a Z-axis of a three-dimensional coordinate system in a real-time manner,
wherein the protective gear further comprises:
a controller coupled with the acceleration sensor and configured to calculate a sum of the acceleration vectors of the part of a human body or the object along the X-axis, the Y-axis and the Z-axis, and determine if the sum of the acceleration vectors of the part of a human body or the object along the X-axis, the Y-axis and the Z-axis is less than or equal to a threshold, and if so, start timing and determine within a continuous preset time period, if the sum of the acceleration vectors of the part of a human body or the object along the X-axis, the Y-axis and the Z-axis is less than or equal to the threshold, and if yes, determine that falling happens and generate an actuating signal to deploy the airbag (5),
wherein the threshold is in a range from 0.6g to 0.9g and g is a gravitational acceleration, and the preset time period is in a range from 100 milliseconds to 300 milliseconds.

2. The protective gear according to Claim 1, wherein the protective gear is configured to cushion a body joint of a human body or a delicate device,
wherein the protective gear is configured to cushion the body joint of the human body, selected from one of elbow, knee, neck, or waist, and the impact happens after falling of the body joint of the human body to ground.

3. The protective gear according to Claim 1, further comprising a gas generator (4), wherein the gas generator (4) is disposed in the housing (1), and is configured to produce gas to inflate and deploy the airbag out of the housing (1) in response to the actuating signal generated by the controller.

4. The protective gear according to Claim 3, wherein the gas generator (4) and the airbag (5) are arranged in an airbag groove (6) in the housing (1); and
an airbag outlet is arranged on an outer surface of the housing (1) opposite to the part of a human body or the object to be cushioned, is spatially connected with the airbag groove, and is configured to allow the airbag to expand out of the airbag groove (6) therethrough upon inflation and deployment by the gas generator.

5. The protective gear according to Claim 3 or 4, wherein the gas generator (4) comprises a tip discharge device and a gas generating agent,
the tip discharge device is configured to generate an electrical spark in response to the actuating signal generated by the controller; and
the gas generating agent is configured to generate gas upon contacting the electrical spark, and
the gas generating agent is selected from a non-azide gas generant or liquid CO₂.

6. The protective gear according to Claim 4, wherein the airbag (5) is detachably mounted in the airbag groove (6), and the airbag groove (6) is provided with a retractable window, arranged on a side wall that is close to the outer surface of the housing (1) and configured to allow replacing the airbag (5) therethrough.

7. The protective gear according to Claim 1, wherein the housing has a ring-shaped structure, encircling the portion of the part of a human body or the object and comprises two ends for assembly or disassembly,
one end is provided with at least one connector;
another end is provided with at least one mating connector; and
each of the at least one connector and each of the at least one mating connector are configured to form a detachable connection between the two ends, and the at least one connector and the at least one mating connector are respectively a plug (9) and a socket (10), or a socket (10) and a plug (9).

8. A protection system for cushioning a part of a human body or an object against falling, comprising a primary protective gear according to claim 1 and at least one secondary protective gear,
wherein the primary protective gear comprises a first communication module coupled with the controller,
wherein the controller is configured to send the actuating signal to deploy the airbag of the primary protective gear to the first communication module; and the first communication module is configured to transmit the actuating signal obtained from the controller; and
wherein each of the at least one secondary protective gear comprises a second communication module and a second airbag, the second communication module is configured to receive the actuating signal from the first communication module of the primary protective gear and to send the actuating signal to deploy the second airbag.

9. The protection system according to Claim 8, wherein the first communication module and the second communication module function in a wireless manner.

10. The protection system according to Claim 9, wherein the part of a human body is a body joint of a human body, the protective gear system comprises four protective gears, two configured to be worn on elbows and another two on knees; and one protective gear is the primary protective gear, and another three protective gears are each one of the at least one secondary protective gear.

11. A method for cushioning a part of a human body or an object against an impact by means of a protective gear according to claim 1, comprising steps of:
detecting acceleration vectors of the part of a human body or the object along an X-axis, a Y-axis and a Z-axis of a three-dimensional coordinate system in a real-time manner;
calculating a sum of the acceleration vectors of the part of a human body or the object along the X-axis, the Y-axis and the Z-axis;
determining if the sum of the acceleration vectors of the part of a human body or the object along the X axis, the Y axis and the Z-axis is less than or equal to a threshold, and if so, starting timing, wherein the threshold is in a range from 0.6g to 0.9g and g is a gravitational acceleration; and
determining in a continuous preset time period, if the sum of accelerations vectors of the part of a human body or the object along the X axis, the Y axis and the Z-axis is less than or equal to the threshold, and if so, determining that falling happens, wherein the preset time period is in a range from 100 milliseconds to 300 millisecond.

## Patentansprüche

1. Schutzausrüstung zum Abfedern eines menschlichen Körperteils oder eines Objekts gegen einen Aufprall,
wobei der Aufprall nach einem Sturz des menschlichen Körperteils oder des Objekts auf den Boden erfolgt, wobei die Schutzausrüstung umfasst:
ein Gehäuse (1), das sich an einen Abschnitt des menschlichen Körperteils oder des Objekts anpasst;
einen Airbag (5), der in dem Gehäuse (1) angeordnet ist;
einen Detektor, der einen Beschleunigungssensor aufweist, der dafür eingerichtet ist,
Beschleunigungsvektoren des menschlichen Körperteils oder des Objekts entlang einer X-Achse, einer Y-Achse und einer Z-Achse eines dreidimensionalen Koordinatensystems in Echtzeit zu erfassen,
wobei die Schutzausrüstung ferner aufweist:
eine Steuereinheit, die mit dem Beschleunigungssensor gekoppelt und dafür eingerichtet ist, eine Summe der Beschleunigungsvektoren des menschlichen Körperteils oder des Objekts entlang der X-Achse, der Y-Achse und der Z-Achse zu berechnen und zu bestimmen, ob die Summe der Beschleunigungsvektoren des menschlichen Körperteils oder des Objekts entlang der X-Achse, der Y-Achse und der Z-Achse kleiner oder gleich einem Schwellenwert ist, und wenn dies der Fall ist, eine Zeitmessung zu starten und innerhalb eines kontinuierlichen voreingestellten Zeitraums zu bestimmen, ob die Summe der Beschleunigungsvektoren des menschlichen Körperteils oder des Objekts entlang der X-Achse, der Y-Achse und der Z-Achse kleiner oder gleich dem Schwellenwert ist, und wenn ja, zu bestimmen, dass ein Sturz vorliegt, und ein Auslösesignal zum Entfalten des Airbags (5) zu erzeugen,
wobei der Schwellenwert in einem Bereich von 0,6 g bis 0,9 g liegt und g die Erdbeschleunigung ist, und der voreingestellte Zeitraum in einem Bereich von 100 Millisekunden bis 300 Millisekunden liegt.

2. Schutzausrüstung nach Anspruch 1, wobei die Schutzausrüstung dafür eingerichtet ist, ein Körpergelenk eines menschlichen Körpers oder einer empfindlichen Vorrichtung abzufedern,
wobei die Schutzausrüstung dafür eingerichtet ist, das Körpergelenk des menschlichen Körpers abzufedern, das aus einem von Ellbogen, Knie, Nacken und Taille ausgewählt ist, und der Aufprall nach dem Sturz des Körpergelenks des menschlichen Körpers auf den Boden erfolgt.

3. Schutzausrüstung nach Anspruch 1, ferner einen Gasgenerator (4) aufweisend,
wobei der Gasgenerator (4) in dem Gehäuse (1) angeordnet ist und dafür eingerichtet ist, als Reaktion auf das Auslösesignal, das von der Steuereinheit erzeugt ist, Gas zum Aufblasen und Entfalten des Airbags aus dem Gehäuse (1) heraus zu erzeugen.

4. Schutzausrüstung nach Anspruch 3, wobei der Gasgenerator (4) und der Airbag (5) in einer Airbag-Nut (6) in dem Gehäuse (1) angeordnet sind; und
ein Airbag-Auslass an einer Außenfläche des Gehäuses (1) gegenüber dem zu schützenden menschlichen Körperteil oder Objekt angeordnet ist, räumlich mit der Airbag-Nut verbunden ist und dafür eingerichtet ist, es dem Airbag zu ermöglichen, sich beim Aufblasen und Entfalten durch den Gasgenerator durch diesen hindurch aus der Airbag-Nut (6) heraus auszudehnen.

5. Schutzausrüstung nach Anspruch 3 oder 4, wobei der Gasgenerator (4) eine Spitzenentladungsvorrichtung und ein Gaserzeugungsmittel aufweist,
die Spitzenentladungsvorrichtung dafür eingerichtet ist, als Reaktion auf das von der Steuereinheit erzeugte Auslösesignal einen elektrischen Funken zu erzeugen; und
das Gaserzeugungsmittel dafür eingerichtet ist, bei Kontakt mit dem elektrischen Funken Gas zu erzeugen, und
das Gaserzeugungsmittel aus einem nicht-azidhaltigen Gaserzeugungsmittel oder flüssigem CO₂ ausgewählt ist.

6. Schutzausrüstung nach Anspruch 4, wobei der Airbag (5) lösbar in der Airbag-Nut (6) angebracht ist und die Airbag-Nut (6) mit einem versenkbaren Fenster versehen ist, das an einer Seitenwand nahe der Außenfläche des Gehäuses (1) angeordnet und dafür eingerichtet ist, einen Austausch des Airbags (5) durch dieses hindurch zu ermöglichen.

7. Schutzausrüstung nach Anspruch 1, wobei das Gehäuse eine ringförmige Struktur aufweist, die den Abschnitt des menschlichen Körperteils oder des Objekts umgibt, und zwei Enden zum Ein- oder Ausbau aufweist,
wobei ein Ende mit mindestens einem Verbinder versehen ist;
ein anderes Ende mit mindestens einem Gegenverbinder versehen ist; und
jeder des mindestens einen Verbinders und jeder des mindestens einen Gegenverbinders dafür eingerichtet sind, eine lösbare Verbindung zwischen den beiden Enden zu bilden, und der mindestens eine Verbinder und der mindestens eine Gegenverbinder jeweils ein Stecker (9) und eine Buchse (10) oder eine Buchse (10) und ein Stecker (9) sind.

8. Schutzsystem zum Abfedern eines menschlichen Körperteils oder eines Objekts bei einem Sturz, umfassend eine primäre Schutzausrüstung nach Anspruch 1 und mindestens eine sekundäre Schutzausrüstung,
wobei die primäre Schutzausrüstung ein erstes Kommunikationsmodul aufweist, das mit der Steuereinheit gekoppelt ist,
wobei die Steuereinheit dafür eingerichtet ist, das Auslösesignal zum Entfalten des Airbags der primären Schutzausrüstung an das erste Kommunikationsmodul zu senden; und das erste Kommunikationsmodul dafür eingerichtet ist, das von der Steuereinheit erhaltene Auslösesignal zu übertragen; und
wobei jede der mindestens einen sekundären Schutzausrüstung ein zweites Kommunikationsmodul und einen zweiten Airbag aufweist, wobei das zweite Kommunikationsmodul dafür eingerichtet ist, das Auslösesignal von dem ersten Kommunikationsmodul der primären Schutzausrüstung zu empfangen und das Auslösesignal zum Entfalten des zweiten Airbags zu senden.

9. Schutzsystem nach Anspruch 8, wobei das erste Kommunikationsmodul und das zweite Kommunikationsmodul drahtlos arbeiten.

10. Schutzsystem nach Anspruch 9, wobei der menschliche Körperteil ein Körpergelenk eines Menschen ist, das Schutzausrüstungssystem vier Schutzausrüstungen aufweist, von denen zwei dafür eingerichtet sind, an den Ellbogen getragen zu werden, und zwei weitere, an den Knien getragen zu werden; und wobei eine Schutzausrüstung die primäre Schutzausrüstung ist und die anderen drei Schutzausrüstungen jeweils eine der mindestens einen sekundären Schutzausrüstungen sind.

11. Verfahren zum Abfedern eines menschlichen Körperteils oder eines hervorstehenden Teils eines Objekts gegen einen Aufprall mittels einer Schutzausrüstung nach Anspruch 1, die folgenden Schritte umfassend:
Erfassen von Beschleunigungsvektoren des menschlichen Körperteils oder des Objekts entlang einer X-Achse, einer Y-Achse und einer Z-Achse eines dreidimensionalen Koordinatensystems in Echtzeit;
Berechnen einer Summe der Beschleunigungsvektoren des menschlichen Körperteils oder des Objekts entlang der X-Achse, der Y-Achse und der Z-Achse;
Bestimmen, ob die Summe der Beschleunigungsvektoren des menschlichen Körperteils oder des Objekts entlang der X-Achse, der Y-Achse und der Z-Achse kleiner oder gleich einem Schwellenwert ist, und, falls ja, Starten einer Zeitmessung, wobei der Schwellenwert in einem Bereich von 0,6 g bis 0,9 g liegt und g die Erdbeschleunigung ist; und
Bestimmen in einem kontinuierlichen, voreingestellten Zeitraum, ob die Summe der Beschleunigungsvektoren des menschlichen Körperteils oder des Objekts entlang der X-Achse, der Y-Achse und der Z-Achse kleiner oder gleich dem Schwellenwert ist, und, falls ja, zu bestimmen, dass ein Sturz vorliegt, wobei der voreingestellte Zeitraum in einem Bereich von 100 Millisekunden bis 300 Millisekunden liegt.

## Revendications

1. Un équipement de protection destiné à amortir une partie du corps humain ou un objet contre un impact, l'impact se produisant après la chute de la partie du corps humain ou de l'objet au sol, l'équipement de protNection comprenant :
un boîtier (1) épousant une portion de la partie du corps humain ou de l'objet ;
un coussin gonflable (5) disposé dans le boîtier (1) ;
un détecteur comprenant un capteur d'accélération configuré pour détecter les vecteurs d'accélération de la partie du corps humain ou de l'objet le long d'un axe X, d'un axe Y et d'un axe Z d'un système de coordonnées tridimensionnel de manière en temps réel,
dans lequel l'équipement de protection comprend en outre :
un contrôleur couplé au capteur d'accélération et configuré pour calculer une somme des vecteurs d'accélération de la partie du corps humain ou de l'objet le long de l'axe X, de l'axe Y et de l'axe Z, et déterminer si la somme des vecteurs d'accélération de la partie du corps humain ou de l'objet le long de l'axe X, de l'axe Y et de l'axe Z est inférieure ou égale à un seuil, et si tel est le cas, démarrer un chronométrage et déterminer dans une période de temps prédéfinie continue,
si la somme des vecteurs d'accélération de la partie du corps humain ou de l'objet le long de l'axe X, de l'axe Y et de l'axe Z est inférieure ou égale au seuil, et si oui, déterminer qu'une chute se produit et générer un signal d'actionnement pour déployer le coussin gonflable (5),
dans lequel le seuil se situe dans une plage de 0,6g à 0,9g et g est une accélération gravitationnelle, et la période de temps prédéfinie se situe dans une plage de 100 millisecondes à 300 millisecondes.

2. L'équipement de protection selon la revendication 1, dans lequel l'équipement de protection est configuré pour amortir une articulation corporelle d'un corps humain ou un dispositif délicat,
dans lequel l'équipement de protection est configuré pour amortir l'articulation corporelle du corps humain, choisie parmi le coude, le genou, le cou ou la taille, et l'impact se produit après la chute de l'articulation corporelle du corps humain au sol.

3. L'équipement de protection selon la revendication 1, comprenant en outre un générateur de gaz (4), dans lequel le générateur de gaz (4) est disposé dans le boîtier (1), et est configuré pour produire du gaz afin de gonfler et de déployer le coussin gonflable hors du boîtier (1) en réponse au signal d'actionnement généré par le contrôleur.

4. L'équipement de protection selon la revendication 3, dans lequel le générateur de gaz (4) et le coussin gonflable (5) sont disposés dans une rainure de coussin gonflable (6) dans le boîtier (1) ; et
une sortie de coussin gonflable est aménagée sur une surface extérieure du boîtier (1) opposée à la partie du corps humain ou à l'objet à amortir, est reliée spatialement à la rainure de coussin gonflable, et est configurée pour permettre au coussin gonflable de se déployer hors de la rainure de coussin gonflable (6) à travers celle-ci lors du gonflage et du déploiement par le générateur de gaz.

5. L'équipement de protection selon la revendication 3 ou 4, dans lequel le générateur de gaz (4) comprend un dispositif de décharge à pointe et un agent générateur de gaz, le dispositif de décharge à pointe est configuré pour générer une étincelle électrique en réponse au signal d'actionnement généré par le contrôleur ; et
l'agent générateur de gaz est configuré pour générer du gaz lors du contact avec l'étincelle électrique, et
l'agent générateur de gaz est choisi parmi un générateur de gaz sans azide ou du CO2 liquide.

6. L'équipement de protection selon la revendication 4, dans lequel le coussin gonflable (5) est monté de manière amovible dans la rainure de coussin gonflable (6), et la rainure de coussin gonflable (6) est pourvue d'une fenêtre rétractable, aménagée sur une paroi latérale qui est proche de la surface extérieure du boîtier (1) et configurée pour permettre le remplacement du coussin gonflable (5) à travers celle-ci.

7. L'équipement de protection selon la revendication 1, dans lequel le boîtier a une structure en forme d'anneau, encerclant la portion de la partie du corps humain ou de l'objet et comprend deux extrémités pour l'assemblage ou le désassemblage, une extrémité est pourvue d'au moins un connecteur ;
l'autre extrémité est pourvue d'au moins un connecteur correspondant ; et
chacun de l'au moins un connecteur et chacun de l'au moins un connecteur correspondant sont configurés pour former une connexion amovible entre les deux extrémités, et l'au moins un connecteur et l'au moins un connecteur correspondant sont respectivement une fiche (9) et une prise (10), ou une prise (10) et une fiche (9).

8. Un système de protection pour amortir une partie d'un corps humain ou un objet contre une chute, comprenant un équipement de protection primaire selon la revendication 1 et au moins un équipement de protection secondaire,
dans lequel l'équipement de protection primaire comprend un premier module de communication couplé au contrôleur,
dans lequel le contrôleur est configuré pour envoyer le signal d'actionnement pour déployer le coussin gonflable de l'équipement de protection primaire au premier module de communication ; et le premier module de communication est configuré pour transmettre le signal d'actionnement obtenu du contrôleur ; et
dans lequel chacun de l'au moins un équipement de protection secondaire comprend un deuxième module de communication et un deuxième coussin gonflable, le deuxième module de communication est configuré pour recevoir le signal d'actionnement du premier module de communication de l'équipement de protection primaire et pour envoyer le signal d'actionnement afin de déployer le deuxième coussin gonflable.

9. Le système de protection selon la revendication 8, dans lequel le premier module de communication et le deuxième module de communication fonctionnent de manière sans fil.

10. Le système de protection selon la revendication 9, dans lequel la partie du corps humain est une articulation corporelle d'un corps humain, le système d'équipement de protection comprend quatre équipements de protection, deux configurés pour être portés sur les coudes et deux autres sur les genoux ; et un équipement de protection est l'équipement de protection primaire, et trois autres équipements de protection sont chacun l'un de l'au moins un équipement de protection secondaire.

11. Une méthode pour amortir une partie d'un corps humain ou un objet contre un impact au moyen d'un équipement de protection selon la revendication 1, comprenant les étapes de :
détection de vecteurs d'accélération de la partie du corps humain ou de l'objet le long d'un axe X, d'un axe Y et d'un axe Z d'un système de coordonnées tridimensionnel de manière en temps réel ;
calcul d'une somme des vecteurs d'accélération de la partie du corps humain ou de l'objet le long de l'axe X, de l'axe Y et de l'axe Z ;
détermination si la somme des vecteurs d'accélération de la partie du corps humain ou de l'objet le long de l'axe X, de l'axe Y et de l'axe Z est inférieure ou égale à un seuil, et si c'est le cas, démarrage de la temporisation, dans lequel le seuil se situe dans une plage de 0,6g à 0,9g et g est une accélération gravitationnelle ; et
détermination dans une période de temps prédéfinie continue, si la somme des vecteurs d'accélération de la partie du corps humain ou de l'objet le long de l'axe X, de l'axe Y et de l'axe Z est inférieure ou égale au seuil, et si c'est le cas, détermination qu'une chute se produit, dans lequel la période de temps prédéfinie se situe dans une plage de 100 millisecondes à 300 millisecondes.
